Europäisches Patentamt

(19) European Patent Office

Office européen des brevets

(11) Numéro de publication : **0 424 318 B1**

## (12) FASCICULE DE BREVET EUROPEEN

(45) Date de publication du fascicule du brevet :
08.06.94 Bulletin 94/23

(21) Numéro de dépôt : **90810779.0**

(22) Date de dépôt : **10.10.90**

(51) Int. Cl.⁵ : **C07K 3/28,** // **A61K37/64**

(54) Procédé de préparation d'inhibiteurs de protéases.

(30) Priorité : **16.10.89 CH 3760/89**

(43) Date de publication de la demande :
**24.04.91 Bulletin 91/17**

(45) Mention de la délivrance du brevet :
**08.06.94 Bulletin 94/23**

(84) Etats contractants désignés :
**AT BE DE DK ES FR GB GR IT NL SE**

(56) Documents cités :
**US-A- 3 983 001**
**PROCEEDINGS OF THE INTERNATIONAL**
**CONFERENCE ON BIOCHEMICAL SEPARA-**
**TIONS 2ND,1988, pages 89-101; K. ZUKLYS et**
**al.: "Human alpha-1-antichymorypsin:purifi-**
**cation by preparative isoelectric focusing, and**
**development of the enzymeimmunoassay"**
**JOURNAL OF CHROMATOGRAPHY, vol. 296,**
**1984, pages 221-229, Elsevier SciencePublis-**
**hers B.V., Amsterdam, NL; G. GUNZER et al.:**
**"Purification of alpha1-**
**proteinase inhibitor by triazine dye affinity**
**chromatography, ion-exchange,chromatogra-**
**phy and gel filtration on fractogel TSK"**
**BIOCHEMISTRY, vol. 13, no. 26, 1974, pages**
**5439-5445; R. PANNELL et al.:"Isolation and**
**properties of human plasma alpha-1-protei-**
**nase inhibitor"**
**PREPARATIVE BIOCHEMISTRY, vol. 5, nos.**
**5,6, 1975, pages 433-453, Marcel Dekker,Inc.;**
**E.J. WHITLEY et al.: "Isolation of alpha1-**
**antitrypsin in a newpreparative electropho-**
**resis apparatus"**
**ELECTROPHORESIS, vol. 4, 1983, pages**
**205-211, Verlag Chemie GmbH, Weinheim,**
**DE;R.C. ALLEN et al.: "Pseudo-ligand affinity**
**chromatography and high-voltageisoelectric**
**focusing as a multiparameter method for**
**separation andidentification of plasma pro-**
**teins"**

(56) Documents cités :
**BIOCHIMICA et BIOPHYSICA ACTA, vol. 797,**
**1984, pages 336-342, Elsevier SciencePublis-**
**hers B.V.; A. PELLEGRINI et al.: "The isolation**
**and characterization of anew elastase inhi-**
**bitor, pre-alpha2-elastase inhibitor, of the hor-**
**se"**
**CHEMICAL ABSTRACTS, vol. 90, no. 17, 23**
**avril 1979, page 325, résumé no.**
**135633n, Columbus, Ohio, US; R. VON FEL-**
**LENBERG: "Electrophoretic analysis ofpro-**
**tease inhibitors in horses serum", &**
**SCHWEIZ. ARCH. TIERHEILKD.1978, 120(12),**
**631-42**

(73) Titulaire : **SEROLAB SA**
**Ch. de la Vulliette 4**
**En Marin**
**Case postale 36**
**CH-1000 Lausanne 25 (CH)**
Titulaire : **Appelboom, Thierry, Prof. Dr.**
**27, Avenue Erasme**
**B-1810 Wemmel (BE)**

(72) Inventeur : **Appelboom, Thierry, Prof. Dr.**
**27, Avenue Erasme**
**B-1810 Wemmel (BE)**

(74) Mandataire : **AMMANN PATENTANWAELTE**
**AG BERN**
**Schwarztorstrasse 31**
**CH-3001 Bern (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a trait à la biochimie et concerne un procédé de préparation d'inhibiteurs de protéases, y compris l'alpha$_1$-antitrypsine, à partir d'un sérum sanguin. Elle concerne également un procédé de ce type appliqué au sérum chevalin comme matière de départ et ayant un résultat inattendu.

Les inhibiteurs de protéases ont acquis une grande importance en pathologie. Le maintien de la santé humaine et animale requiert une balance entre les systèmes offensifs et défensifs dans le corps d'un être vivant. Une telle balance récemment découverte est celle entre les enzymes qui sont les biocatalyseurs de la scission des protéines, donc les protéases, et les antiprotéases qui inhibent l'action de ces enzymes. Des détails se rapportant à ces balances biomoléculaires ont été décrits par R.W. Carrel dans J. Clin. Invest. $\underline{78}$, 1427-31 (1986).

Un représentant important des inhibiteurs des protéases est l'alpha$_1$-antitrypsine (alpha$_1$-AT). Elle appartient à une famille d'inhibiteurs de protéases appelée serpines, et elle est synthétisée par les hépatocytes, les macrophages, et les monocytes. L'alpha$_1$-antitrypsine est un inhibiteur des sérine-protéases, qui représentent le plus grand groupe des enzymes chez les mammifères.

Elle est un inhibiteur majeur des enzymes suivantes: élastase, catepsine G, collagénase, trypsine, chymotrypsine, facteur XIa, urokinase. Une déficience en alpha$_1$-AT a été retrouvée chez certains malades souffrant de maladies telles que la polyarthrite rhumatoide, l'uvéite antérieure et le lupus erythémateux, et on a montré que l'alpha$_1$-AT pouvait être importante non seulement en tant que protéine anti-inflammatoire mais aussi en tant qu'immunomodulateur.

Selon la demande de brevet EP-A2-67 293, on utilise comme matière de départ du plasma sanguin humain. On réduit chimiquement les liaisons disulfure des protéines contaminantes, et l'on obtient après tout un nombre d'étapes successives, une purification de l'anti-enzyme par un facteur de 15,3 avec un rendement de 50%.

L'article "Human alpha-1-antichymotrypsin: Purification by preparative isoelectric focusing, and development of the enzyme immunoassay" par K. Zuklys et al., Proceedings of the International Conference on Biochemical Separations, 2nd., 1988, p. 89-101 se réfère à la purification de l'antienzyme indiquée ci-dessus et décrit la séparation de la séroalbumine par chromatographie d'affinité puis le traitement de l'éluat par focalisation isoélectrique en couche plate ("Multiphor flat-bed IEF", donc sur plaque de gel). Dans le US-A-3,983,001, on isole l'inhibiteur de chymotrypsine à partir de pommes de terre, et l'antitrypsine à partir du soja par chromatographie d'affinité; on ne mentionne pas de focalisation isoélectrique.

Le but de l'invention est de trouver un procédé comparativement plus simple, nécessitant moins d'étapes par rapport aux procédés connus, qui permette de préparer un mélange d'inhibiteurs de protéases, par exemple un mélange contenant les facteurs de l'alpha$_1$-AT et, si désiré, chacun des facteurs du mélange en quantités appréciables et d'une pureté acceptable pour une utilisation en pharmacie ou cosmétique.

Le procédé qui permet d'atteindre ce but est défini dans la première revendication indépendante. Des modes d'exécution préférés font l'objet des revendications dépendantes.

La première étape de ce procédé comprend la purification du sérum qui est utilisé comme produit de départ. On peut utiliser le sérum juste après son obtention. Il est également possible d'utiliser le sérum reconstitué obtenu par dissolution du sérum lyophilisé dans une solution saline physiologique, ou de préférence dans une solution tampon, par exemple un tampon au phosphate. Cette solution est de préférence dialysée pendant un certain temps pour enlever des sels indésirables.

La prochaine étape comprend la séparation sélective de l'albumine de la solution tamponnée éventuellement dialysée. Il est à noter que, contrairement aux procédés connus où la plupart des protéines sont précipitées, entraînant nécessairement une certaine perte d'antiprotéases, le procédé de l'invention se contente d'enlever l'albumine. On utilise pour cette séparation de préférence un procédé de chromatographie d'affinité en colonne comportant un gel adsorptif. Il s'agit en l'espèce de l'agarose coloré par du Coomassie (ICI), un colorant bleu réactif à anneau triazine. Cet agarose retient l'albumine à 100% mais laisse passer les autres protéines. La colonne peut facilement être régénérée avec une solution de chlorure de sodium.

L'étape suivante est la séparation des protéines restantes par concentration isoélectrique ("isoelectrofocusing"). Cette méthode est basée sur le phénomène que les protéines, sous l'influence d'un champ électrique superposé à un gradient de pH, migrent dans la zone où le pH est égal au pI (point isoélectrique) de protéine. Pour mettre en pratique cette méthode, il existe depuis peu de temps un appareil appelé "Rotofor®", commercialisé par la société Bio-Rad, Richmond, CA (USA). Cet appareil permet la concentration de plus de 25 fois, avec séparation simultanée, de chaque protéine d'un mélange après 4 heures seulement, et il comprend 20 chambres pour 20 fractions.

Ce qui importe dans le procédé de l'invention est la méthode de concentration isoélectrique préparative quasiment tridimensionnelle et non pas l'utilisation d'un appareil particulier, bien que le Rotofor donne des résultats appropriés.

De préférence, avant de soumettre la solution de protéines exempte d'albumine à la concentration isolélectrique préparative, on lui fait subir une dialyse rapide contre l'eau distillée ou déminéralisée, ou bien une chromatographie, afin d'enlever la plupart des sels du tampon. Un liquide à concentrer isoélectriquement ne doit pas présenter une conductivité trop élevée afin qu'un champ électrique de quelques 20 à 80 V/cm puisse s'établir. La solution est séparée en plusieurs échantillons, chacun est dilué avec un ampholyte ayant un pH différent, et l'échantillon est introduit dans l'appareil, chacun dans une cellule séparée, en observant un gradient unidirectionnel du pH. Après concentration isoélectrique, les cellules sont vidées, chacune dans un récipient séparé.

Ensuite, les fractions acides ayant un pH compris entre 3 et 5 environ, plus spécialement entre 3,5 et 4,0, sont travaillées pour isoler le mélange d'antiprotéases intéressé. Les inhibiteurs peuvent alors être séparés des ampholytes solubilisants par des méthodes connues en soi. Il a été trouvé que le procédé de l'invention prévoit la séparation par chromatographie d'affinité en colonne sur un agarose comportant une protéase liée, par exemple l'élastase. Lors de la chromatographie, les inhibiteurs se fixent sur les protéases immobilisées, et ils peuvent être récupérés par élution en utilisant une solution ayant un autre potentiel ionique - changement du pI - et/ou une molarité différente de sels.

On peut finalement séparer le mélange d'inhibiteurs obtenu selon le poids moléculaire, pour obtenir les facteurs du mélange séparément. Une des méthodes de séparation est alors la chromatographie liquide à moyenne ou haute performance en phase renversée (MPLC ou HPLC, respectivement); une autre en est l'électrophorèse 1x1 bidimensionnelle sur gel.

Il a été trouvé que le procédé selon l'invention permet la préparation à partir d'un sérum humain d'un mélange d'inhibiteurs de protéase contenant quatre inhibiteurs individuels au moins, à savoir l'alpha$_1$-antichymotrypsine, l'alpha$_1$-antitrypsine, l'antithrombine III, et le dimère de l'antitrypsine/ antichymotrypsine. Le mélange contient encore une alpha$_1$-glycoprotéine, du fibrinogène, et d'autres protéines en quantités négligeables. L'activité du mélange et des différents facteurs à été déterminée par l'action sur l'élastase.

Il a encore été trouvé qu'un nouvel inhibiteur de protéases, inconnu jusqu'à ce jour, est obtenu lorsqu'on applique le procédé de l'invention au sérum chevalin et qu'on fractionne le mélange d'inhibiteurs finalement obtenu. Ce nouvel inhibiteur exerce une action inhibitrice sur l'élastase et présente un poids moléculaire d'environ 38 kD (kilodalton) et un point isoélectrique d'environ 4,2. Cet inhibiteur est absent dans le sérum humain. Il est appelé provisoirement "horse inhibitor" d'après sa provenance.

L'invention sera expliquée plus en détail par un exemple d'exécution.

## 1. - Préparation de la matière première

Du sérum chevalin lyophilisé est solubilisé, à raison de 1,5 g dans 5 ml de liquide, dans un tampon au phosphate 50 mM, pH 8 (PBS).

## 2. - Chromatographie d'affinité

Une colonne contenant de l'agarose bleu (colorant réactif triaziné "Coomassie" ou "CIBACRON® Blue F3GA" lié à l'agarose) est équilibrée avec 50 ml de PBS.

Le sérum reconstitué et tamponné est chromatographié sur la colonne ce qui a pour résultat l'enlèvement à pratiquement 100% de l'albumine sans altération des autres protéines.

La colonne peut être régénérée par une solution 2M de NaCl et rinçage extensif avec PBS 50 mM.

## 3. - Concentration isoélectrique sur Rotofor

a. On effectue une dialyse rapide au cours de 3 à 4 heures contre l'eau distillée des effluents de la colonne d'affinité. On dilue ensuite les effluents dialysés réunis à un volume de 50 ml avec des solutions aqueuses d'ampholytes concentrés de 40%, diluées à 2% en poids.

b. Ces solutions aqueuses contiennent alors chacune 2% en poids d'un ampholyte dans la gamme des points isoélectriques de 3 à 10. Il a été trouvé avec surprise que la concentration normale et recommandée de 1% d'ampholyte ne donne pas satisfaction, et qu'une augmentation de cette concentration à 2% a fourni une séparation satisfaisante. De même, les concentrations isoélectriques de protéines semblables se font normalement avec les ampholytes d'un pI de 5 à 7, et non de 3 à 10.

c. L'étape de la concentration isoélectrique à été conduite pendant 4 heures avec une puissance de 12 W. L'appareil est arrêté lorsqu'un plafond de voltage de 1000 à 1200 V est atteint. Au cours du travail, le contenu de toutes les cellules du Rotofor est maintenu à une température de 3°C environ.

d. L'appareil est vidé, chaque cellule dans un récipient séparé, et les protéines sont séparées de l'ampholyte par une dialyse poussée contre une solution 2M de NaCl; ensuite contre 1M PBS, et les protéines ayant un pI de 3,5 à 4,0 ont été récupérées.

4. - Séparation des protéines à partir des ampholytes

On a préparé une colonne d'agarose dans laquelle on a lié de l'élastase ou une autre protéase à l'agarose. Les fractions acides obtenues dans l'étape de concentration isoélectrique dans lesquelles une activité d'antiprotéase avait été trouvée, ont été filtrées séparément par cette colonne. Les antienzymes ont été réversiblement retenues, et on les a récupérées en éluant avec un tampon de pouvoir ou concentration ioniques différent.

Le contenu de chaque fraction a été finalement contrôlé pour l'activité antiprotéase par une électrophorèse bidimensionnelle.

5. - Détermination de l'activité inhibitrice d'enzymes

L'électrophorèse bidimensionnelle est opérée sur du gel de polyacrylamide, d'abord dans une direction, puis dans une seconde direction perpendiculaire. On procède à une électroélution, et l'on mesure, par exemple par l'activité d'antiélastase, l'hydrolyse d'un substrat de polypeptide comprenant 5 acides aminés, additionné d'élastase (SICMA E.0258). On compare l'hydrolyse du substrat avec et sans addition de la fraction à analyser.

6. - Détermination du poids moléculaire des facteurs

On soumet la fraction à déterminer à une électrophorèse 1x1 bidimensionnelle, et l'on compare les taches obtenues par silver staining avec celles d'un mélange de substances à poids moléculaire et à point isoélectrique connus, en utilisant les mêmes conditions. On obtient à la fois le poids moléculaire et le point isoélectrique exact de la fraction à tester.

Une activité pour inhiber l'élastase a été trouvée pour les fractions 1 à 5 parmi les 20 fractions sortant du Rotofor, correspondant à un pI compris entre 3,5 et 4,0. Ces cinq fractions ont été réunies et analysées plus en détail. Une activité inhibitrice a aussi été constatée concernant la trypsine, la chymotrypsine, la plasmine et la collagénase.

La fraction acide combinée peut être séparée en protéines individuels par électrophorèse sur gel de polyacrylamide uni et bidimensionnelle. On a trouvé 5 protéines majeures ayant des poids moléculaires compris entre 46 et 68 kD. Le degré de purification est supérieur aux préparations d'antienzymes connues. Parmi ces 5 protéines majeures, appelées facteurs, les facteurs 2 et 3 ont été trouvés pour avoir une activité inhibitrice selon le tableau suivant:

### Tableau: Activité inhibitrice des facteurs

| facteur no. | poids mol. | pI | nature |
|---|---|---|---|
| 2 | 55 kD | 3,9 | $alpha_1$-antichymotrypsine |
| 3 | 53 kD | 4,0 | $alpha_1$-antitrypsine et antithrombine III |

Si l'on répète cet exemple, mais en partant du sérum chevalin comme matière de départ, on obtient finalement un sixième facteur, poids moléculaire 38 kD, pI 4,2, actif contre l'élastase, qu'on a appelé "facteur cheval". Ce facteur n'a pas encore été décrit et ne se trouve pas dans le sérum humain.

**Revendications**

1.  Procédé de préparation d'inhibiteurs de protéases à partir d'un sérum sanguin, dont la séroalbumine est enlevée sélectivement par chromatographie d'affinité, caractérisé par les étapes suivantes:

(1) fractionnement du sérum débarrassé de l'albumine par concentration isoélectrique dans un champ électrique à l'intérieur d'une multitude de cellules rotatives adjacentes, séparées les unes des autres par une membrane sélectivement perméable, au sein d'une série d'ampholytes, et
(2) séparation des antiprotéases classées par points isoélectriques, de l'ampholyte par chromatographie d'affinité.

2. Procédé selon la revendication 1, caractérisé en ce qu'on utilise un sérum frais.

3. Procédé selon la revendication 2, caractérisé en ce qu'on purifie d'abord le sérum frais par dialyse.

4. Procédé selon la revendication 1, caractérisé en ce qu'on utilise un sérum lyophilisé reconstitué par dispersion dans une solution aqueuse tampon.

5. Procédé selon l'une des revendications précédentes, caractérisé en ce qu'on sépare sélectivement la séroalbumine du sérum par chromatographie sur une colonne d'agarose bleu comprenant un colorant réactif lié à l'agarose.

6. Procédé selon l'une des revendications précédentes, caractérisé en ce qu'on opère l'étape (1) dans un appareil Rotofor en observant un plage de points isoélectriques compris entre 3 et 10, et qu'on récupère les fractions acides ayant une plage de points isoélectriques compris entre 3,5 et 4,0.

7. Procédé selon l'une des revendications précédentes, caractérisé en ce qu'on sépare les inhibiteurs de protéases contenus dans les fractions à points isoélectriques de 3,5 et 4,0 des ampholytes utilisés par chromatographie, sur une colonne comprenant un enzyme en phase stationnaire, et que l'on élue la colonne en faisant changer les conditions ioniques de l'éluant par rapport à celles des fractions avant la chromatographie.

8. Procédé selon la revendication 1, caractérisé en ce qu'on utilise le sérum humain comme matière de départ, et qu'on obtient un mélange d'inhibiteurs de protéases comprenant cinq facteurs à activité différente.

9. Procédé selon la revendication 1, caractérisé en ce qu'on utilise le sérum chevalin comme matière de départ, et qu'on obtient un mélange d'inhibiteurs de protéases comportant six facteurs à activité différente dont un facteur n'étant présent que dans le sérum chevalin.

10. Procédé selon la revendication 8 ou 9, caractérisé en ce qu'on sépare les différents facteurs du mélange obtenu.

11. Procédé selon la revendication 10, caractérisé en ce qu'on utilise la chromatographie en gel uni- et bidimensionnelle pour la séparation.

12. Facteur inhibiteur de protéases chevalin, obtenu par le procédé selon la revendication 9 et l'une des revendications 10 et 11, présentant un poids moléculaire de 38 kD et un point isoélectrique pI de 4,2.

**Patentansprüche**

1. Verfahren zur Herstellung von Protease-Inhibitoren ausgehend von einem Blutserum, dessen Seroalbumin mittels Affinitäts-Chromatographie selektiv entfernt wurde, gekennzeichnet durch die folgenden Schritte:
(1) Fraktionierung des vom Albumin befreiten Serums, eingebettet in eine Reihe von Ampholyten, durch isoelektrische Aufkonzentrierung in einem elektrischen Feld im Inneren einer Mehrzahl von benachbarten Drehzellen, die voneinander durch eine selektiv durchlässige Membran getrennt sind, und
(2) Trennung der nach isoelektrischen Punkten geordneten Antiproteasen vom Ampholyt durch Affinitäts-Chromatographie.

2. Verfahren nach Anspruch 1, dadurch gekennzeichnet, dass frisches Serum verwendet wird.

3. Verfahren nach Anspruch 2, dadurch gekennzeichnet, dass das frische Serum zunächst durch Dialyse gereinigt wird.

4. Verfahren nach Anspruch 1, dadurch gekennzeichnet, dass ein gefriergetrocknetes und durch Dispergierung in einer wässrigen Pufferlösung wiedergebildetes Serum verwendet wird.

5. Verfahren nach einem der vorstehenden Ansprüche, dadurch gekennzeichnet, dass das Seroalbumin durch Chromatographie an einer Säule mit blauer Agarose, die einen an die Agarose gebundenen Reaktivfarbstoff enthält, selektiv vom Serum getrennt wird.

6. Verfahren nach einem der vorstehenden Ansprüche, dadurch gekennzeichnet, dass der Schritt (1) in einem Rotofor-Apparat ausgeführt wird, in dem ein Bereich der isoelektrischen Punkte zwischen 3 und 10 eingestellt wird, und dass die sauren Fraktionen gewonnen werden, deren isoelektrische Punkte zwischen 3,5 und 4,0 liegen.

7. Verfahren nach einem der vorstehenden Ansprüche, dadurch gekennzeichnet, dass die Protease-Inhibitoren in den Fraktionen mit isoelektrischen Punkten zwischen 3,5 und 4,0 durch Chromatographie an einer Säule, die ein Enzym in stationärer Phase enthält, von den verwendeten Ampholyten getrennt werden. und dass die Säule durch Änderung der Ioneneigenschaften des Eluierungsmittels gegenüber denjenigen der Fraktionen vor der Chromatographie eluiert wird.

8. Verfahren nach Anspruch 1, dadurch gekennzeichnet, dass menschliches Serum als Ausgangsmaterial verwendet wird, und dass ein Gemisch von Protease-Inhibitoren, enthaltend fünf Faktoren mit unterschiedlicher Wirkung, erhalten wird.

9. Verfahren nach Anspruch 1, dadurch gekennzeichnet, dass das Serum von Pferden als Ausgangsmaterial verwendet wird, und dass ein Gemisch von Protease-Inhibitoren, enthaltend sechs Faktoren mit unterschiedlicher Wirkung, erhalten wird, wovon ein Faktor nur im Pferdeserum vorkommt.

10. Verfahren nach Anspruch 8 oder 9, dadurch gekennzeichnet, dass die einzelnen Faktoren aus dem erhaltenen Gemisch abgetrennt werden.

11. Verfahren nach Anspruch 10, dadurch gekennzeichnet, dass zur Trennung die ein- oder zweidimensionale Gelchromatographie angewandt wird.

12. Pferdefaktor-Protease-Inhibitor, erhalten nach dem Verfahren gemäss Anspruch 9 und einem der Ansprüche 10 und 11, mit einem Molekulargewicht von 38 kD und einem isoelektrischen Punkt pI von 4,2.

## Claims

1. Process for the preparation of protease inhibitors from a blood serum where the seroalbumin has selectively been removed by affinity chromatography, characterised by the following steps:
   (1) fractionation of the albumin free serum by isoelectric concentration within an electric field in the interior of a plurality of adjacent rotary cells, separated from each other by a selectively permeable membrane, within a series of ampholytes, and
   (2) separation of the antiproteases classified by isoelectric points from the ampholyte by affinity chromatography.

2. Process according to claim 1, characterised by the fact that fresh serum is used.

3. Process according to claim 2, characterised by the fact that the fresh serum is first purified by dialysis.

4. Process according to claim 1, characterised by the fact that freeze dried serum is used which has been reconstituted by dispersion in an aqueous buffer solution.

5. Process according to any one of the preceding claims, characterised by the fact that the seroalbumin is selectively separated from the serum by chromatography in a blue agarose column containing a reactive dye-stuff bound to the agarose.

6. Process according to any one of the preceding claims, characterised by the fact that the step (1) is performed in a Rotofor apparatus, in applying a range of isoelectric points within 3 and 10, and acidic fractions are recovered having a range of isoelectric points within 3.5 and 4.0.

7.  Process according to any one of the preceding claims, characterised by the fact that the protease inhibitors contained in the fractions having isoelectric points of from 3.5 to 4.0, are separated from the ampholytes used by chromatography in a column comprising an enzyme in stationary phase, and that the column is eluted in changing the ionic conditions of the eluant with respect to those of the fraction before chromatography.

8.  Process according to claim 1, characterised by the fact that human serum is used as a starting material, and a mixture of protease inhibitors is obtained comprising five factors having different activity.

9.  Process according to claim 1, characterised by the fact that horse serum is used as a starting material, and a mixture of protease inhibitors is obtained comprising six factors having different activity, one factor being present in horse serum only.

10.  Process according to claim 8 or 9, characterised by the fact that the different factors are separated from the mixture obtained.

11.  Process according to claim 10, characterised by the fact that unidirectional or bi-directional gel chromatography is used for the separation.

12.  Protease inhibitor horse factor, obtained by the process of claim 9 and any one of claims 10 and 11, having a molecular weight of 38 kD and an isoelectric point pI of 4.2.